# DEMANDE DE BREVET EUROPEEN

(11) **EP 1 462 091 A1**
(43) Date de publication de la demande: **29.09.2004**
(21) Numéro de dépôt: 04290711.3
(22) Date de dépôt: 16.03.2004
(51) Int. Cl.: A61K 7/13, C07D 243/08, C07D 295/12

(54) **Composition de teinture des fibres kératiniques comprenant un dérivé de para-phénylènediamine cationique substitué par un cycle diazacyclohexane ou diazacycloheptane**

(30) Priorité: 24.03.2003 FR 0303547
(71) Demandeur: L'OREAL, 75008 Paris (FR)
(72) Inventeur: Genet, Alain, 93600 Aulnay Sous Bois (FR); Sabelle, Stéphane, 75005 Paris (FR)
(74) Mandataire: Fevrier, Murielle

(57) **Abrégé**

L'invention a pour objet une composition de teinture des fibres kératiniques, en particulier des fibres kératiniques humaines telles que les cheveux, comprenant à titre de base d'oxydation au moins un dérivé de paraphénylènediamine substitué par un cycle diazacyclohexane ou diazacycloheptane dont l'azote en position 4 est un ammonium quaternaire.

La présente invention permet en particulier d'obtenir une coloration des fibres kératiniques chromatique, puissante, peu sélective et tenace.

## Description

L'invention a pour objet une composition de teinture des fibres kératiniques, en particulier des fibres kératiniques humaines telles que les cheveux, comprenant à titre de base d'oxydation au moins un dérivé de paraphénylènediamine substitué par un cycle diazacyclohexane ou diazacycloheptane dont l'azote en position 4 est un ammonium quaternaire.

Il est connu de teindre les fibres kératiniques et en particulier les fibres kératiniques humaines telles que les cheveux avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4-triazole, des dérivés de pyrazolo[3,2-c]-1,2,4-triazole, des dérivés de pyrazolo[1,5-a]pyrimidine, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la paraphénylènediamine et la para-toluènediamine sont des bases d'oxydation largement utilisées. Elles permettent d'obtenir avec des coupleurs d'oxydation des nuances variées.

Cependant, il existe un besoin de découvrir de nouvelles bases d'oxydation présentant un meilleur profil toxicologique que la paraphénylènediamine et la para-toluènediamine, tout en permettant de conférer aux cheveux d'excellentes propriétés d'intensité de couleur, de variété de nuances, d'uniformité de la couleur et de la ténacité aux agents extérieurs.

Il est déjà connu d'utiliser des dérivés 1,4-diazacycloheptane pour la coloration d'oxydation des fibres kératiniques. Par exemple, le brevet US 6 165 230 décrit une composition tinctoriale comprenant des dérivés 1,4-diazacycloheptane substitués sur les deux atomes d'azote du cycle par un groupement 4'-aminobenzène.

Il a déjà été proposé, notamment dans les brevets FR 9 709 027 et FR 9 709 028, d'utiliser des dérivés de para-phénylènediamine comprenant un groupement cationique.

Il est clairement établi que ces composés ne permettent pas de conférer une coloration de qualité équivalente à celle obtenue avec la paraphénylènediamine ou avec la para-toluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions tinctoriales les comprenant permettent de conférer aux cheveux d'excellentes propriétés d'intensité de couleur, de variété de nuances, d'uniformité de la couleur et de ténacité vis-à-vis des différentes agressions extérieures que peuvent subir les cheveux.

Le but de la présente invention est de développer de nouvelles compositions tinctoriales ne présentant pas les inconvénients des bases d'oxydation de la technique antérieure en fournissant de nouvelles compositions tinctoriales pour la teinture des fibres kératiniques qui ne dégradent pas les fibres kératiniques tout en étant capables d'engendrer des colorations intenses dans des nuances variées, peu sélectives, particulièrement résistantes et présentant un bon profil toxicologique.

Ce but est atteint avec la présente invention qui a pour objet une composition tinctoriale des fibres kératiniques, en particulier des fibres kératiniques humaines telles que les cheveux, comprenant, dans un milieu cosmétique approprié à la teinture, à titre de base d'oxydation, au moins un dérivé de para-phénylènediamine de formule (I) ou un de ses sels d'addition : 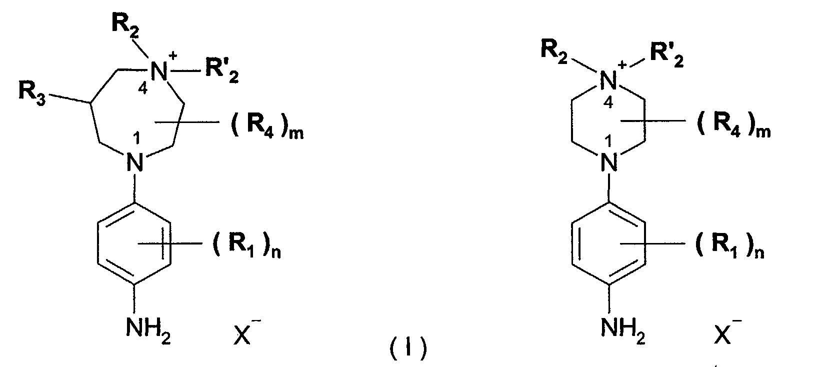 dans laquelle :
- R₁ représente :
   - un atome d'halogène ;
   - une chaîne hydrocarbonée en C₁-C₈, aliphatique ou alicyclique, saturée ou insaturée, un ou plusieurs atomes de carbone pouvant être remplacés par un ou plusieurs atomes d'oxygène, d'azote, de silicium, de soufre ou groupements SO₂ ; le radical R₁ ne comportant pas de liaison peroxyde, ni de radicaux diazo, nitro ou nitroso ;
- n est compris entre 0 et 4, étant entendu que lorsque n est supérieur à 1 alors les radicaux R₁ peuvent être identiques ou différents ;
- R₂ et R'₂, identiques ou différents, représentent :
   - un radical alkyle pouvant être insaturé, non substitué ou substitué par un ou plusieurs radicaux carboxyle, alkylcarbonyle, alcoxycarbonyle, carbamoyle, mono- ou di-alkylcarbamoyle, hétérocycliques à 4, 5, 6 ou 7 atomes, saturés ou insaturés, dont le ou les hétéroatomes sont choisis parmi l'azote, l'oxygène et le soufre ;
   - un radical -CH₂ R où R est un radical alkyle pouvant être insaturé, substitué par un ou plusieurs radicaux hydroxy, alcoxy, cyano, hydrogénothio, halogéno, amino, mono- ou di-alkylamino, amino avec l'amine substituée par un radical alkylcarbonyle, carbamyle ou alkylsulfonyle ;
   - un radical aryle ;
   - un radical benzyle ;
- R₃ représente :
   - un atome d'hydrogène ;
   - un radical alkyle pouvant être insaturé ;
   - un radical hydroxy ;
   - un radical hydroxyalkyle ;
   - un radical alcoxy ;
   - un radical alcoxyalkyle ;
   - un radical alkylcarbonyle ;
   - un radical hydroxyalcoxyalkyle ;
   - un radical amino, l'amine pouvant être mono ou disubstituée par un radical alkyle, acétyle ou hydroxyalkyle ;
   - un radical monoalkylamino ou dialkylamino ;
   - un radical aminoalkyle, l'amine pouvant être mono ou disubstitué par un radical alkyle, acétyle ou hydroxyalkyle ;
   - un radical hydroxy- et amino- alkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono- ou dialkyl- aminocarbonyle ;
   - un radical mono- ou dialkyl- aminocarbonylalkyle ;
- R₄ représente :
   - un radical alkyle pouvant être insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine pouvant être mono ou disubstitué par un radical alkyle, acétyle, hydroxyalkyle ;
   - un radical hydroxy- et amino- alkyle ;
   - un radical carboxyle ;
   - un radical carboxyalkyle ;
   - un radical carbamoyle ;
   - un radical carbamoylalkyle ;
   - un radical alcoxycarbonyle ;
   - un radical mono- ou dialkyl- aminocarbonyle ;
   - un radical mono- ou dialkyl- aminocarbonylalkyle ;
- m est compris entre 0 et 4, étant entendu que lorsque m est supérieur à 1 alors les radicaux R₄ peuvent être identiques ou différents ;
- X⁻ représente un contre-ion.

La présente invention permet en particulier d'obtenir une coloration des fibres kératiniques chromatique, puissante, peu sélective et tenace.

Un autre objet de l'invention est l'utilisation de ces dérivés de formule (I) pour la teinture des fibres kératiniques ainsi que le procédé de teinture des fibres kératiniques mettant en oeuvre la composition de la présente invention.

L'invention a aussi pour objet de nouveaux dérivés de paraphénylènediamine substitués par un cycle diazacyclohexane ou diazacycloheptane dont l'azote en position 4 est un ammonium quaternaire.

L'invention a également pour objet les dérivés de para-nitroaniline substitués par un cycle diazacyclohexane ou diazacycloheptane dont l'azote en position 4 est un ammonium quaternaire permettant la synthèse des dérivés de formule (I) décrits ci-dessus.

Les composés de formule (I) sont des para-phénylènediamines dont une amine est comprise dans un cycle de type 1,4-diazacycloheptane, cycle aussi appelé dans la littérature 1,4-diazépane, ou dont une amine est comprise dans un cycle de type 1,4-diazacyclohexane aussi appelé dans la littérature 1,4-pipérazine.

Dans les définitions ci-dessus, les radicaux alkyle sont linéaires ou ramifiés et comprennent, sauf autre indication, de 1 à 10 atomes de carbone, de préférence de 1 à 6 atomes de carbone. Un radical alcoxy est un radical alkyl-O-, le radical alkyle étant tel que défini précédemment.

On entend par radical alkyle insaturé, un alkyle de 2 à 10 atomes de carbone et comprenant une ou plusieurs doubles et / ou triples liaisons. Un radical alkyle substitué est un alkyle mono ou polysubstitué. Par exemple, un hydroxyalkyle ou un aminoalkyle est un alkyle qui peut être substitué par un ou plusieurs groupes hydroxy ou amino.

Dans les formules (I), le radical R₁ est par exemple choisi parmi un atome de chlore ; un radical méthyle ; un radical éthyle ; un radical isopropyle ; un radical vinyle ; un radical allyle ; un radical méthoxyméthyle ; un radical hydroxyméthyle ; un radical 1-carboxyméthyle ; un radical 1-aminométhyle ; un radical 2-carboxyéthyle ; un radical 2-hydroxyéthyle ; un radical 3-hydroxypropyle ; un radical 1,2-dihydroxyéthyle ; un radical 1-hydroxy-2-aminoéthyle ; un radical 1-amino-2-hydroxyéthyle ; un radical 1,2-diaminoéthyle ; un radical méthoxy ; un radical éthoxy ; un radical allyloxy ; un radical 2-hydroxyéthyloxy.

Selon un mode de réalisation particulier de l'invention, n est égal à 0 ou R₁ est choisi parmi un radical alkyle ; un radical hydroxyalkyle ; un radical aminoalkyle ; un radical alcoxy ; un radical hydroxyalcoxy. Dans ce dernier cas, le radical R₁ peut être un radical méthyle ; un radical hydroxyméthyle ; un radical 2-hydroxyéthyle ; un radical 1,2-dihydroxyéthyle ; un radical méthoxy ; un radical isopropyloxy ; un radical 2-hydroxyéthoxy et plus préférentiellement un radical méthyle ; un radical hydroxyméthyle ; un radical 1,2-dihydroxyéthyle.

Selon un mode de réalisation préféré de l'invention, les bases d'oxydation de formule (I) sont telles que n est égal à 0 ou à 1.

Selon un mode de réalisation particulier de l'invention, le radical R₂ est choisi parmi un radical alkyle ; un radical alkyle substitué par un radical hétérocyclique à 4, 5, 6 ou 7 atomes, saturés ou insaturés, dont le ou les hétéroatomes sont choisis parmi l'azote, l'oxygène et le soufre ; un radical -CH₂R où R est un radical alkyle substitué par un ou plusieurs radicaux hydroxy. Dans ce cas, le radical R₂ est choisi de préférence parmi un radical 2-hydroxyéthyle ; un radical 2,3-dihydroxypropyle ; un radical 3-(1-pyrrolidinyl)propyle ; un radical méthyle et plus préférentiellement un radical 2-hydroxyéthyle et un radical méthyle.

Selon un mode de réalisation particulier de l'invention, le radical R'₂ est choisi parmi un radical alkyle ; un radical -CH₂R où R est un radical alkyle substitué par un ou plusieurs hydroxy, amino, mono ou dialkylamino. Dans ce cas, le radical R'₂ est choisi de préférence parmi un radical méthyle ; un radical éthyle ; un radical 2-hydroxyéthyle.

Selon un mode de réalisation particulier de l'invention, le radical R₃ est choisi parmi un atome d'hydrogène ; un radical alkyle ; un radical hydroxyalkyle ; un radical aminoalkyle ; un radical carboxyle ; un radical carbamoyle ; un radical hydroxyle ; un radical alcoxy ; un radical amino ; un radical mono ou dialkylamino. Dans ce cas, le radical R₃ est choisi parmi un atome d'hydrogène ; un radical hydroxyle ; un radical carboxyle ; un radical carbamoyle ; un radical amino ; un radical hydroxyméthyle ; un radical aminométhyle et représente plus préférentiellement un atome d'hydrogène.

Selon un mode de réalisation particulier de l'invention, m est égal à 0 ou R₄ est choisi parmi un radical alkyle ; un radical hydroxyalkyle ; un radical aminoalkyle ; un radical carboxyle ; un radical carbamoyle ; un radical mono ou dialkylcarbamoyle. De préférence, m est égal à 0 ou 1.

Les composés de formule (I) peuvent être éventuellement salifiés par des acides minéraux forts tels que par exemple HCl, HBr, H₂SO₄, ou des acides organiques tels que, par exemple, l'acide acétique, lactique, tartrique, citrique ou succinique.

A titre d'exemples de contre-ions X⁻, on peut citer les ions halogénure tel que l'ion chlorure, bromure, fluorure ou l'iodure, l'ion hydroxyde, l'ion hydrogènesulfate, les ions alkylsulfate en C₁-C₆ tel que le méthylsulfate ou l'éthylsulfate.

A titre d'exemples des dérivés de formule (I), on peut citer les composés présentés dans le tableau ci-dessous.

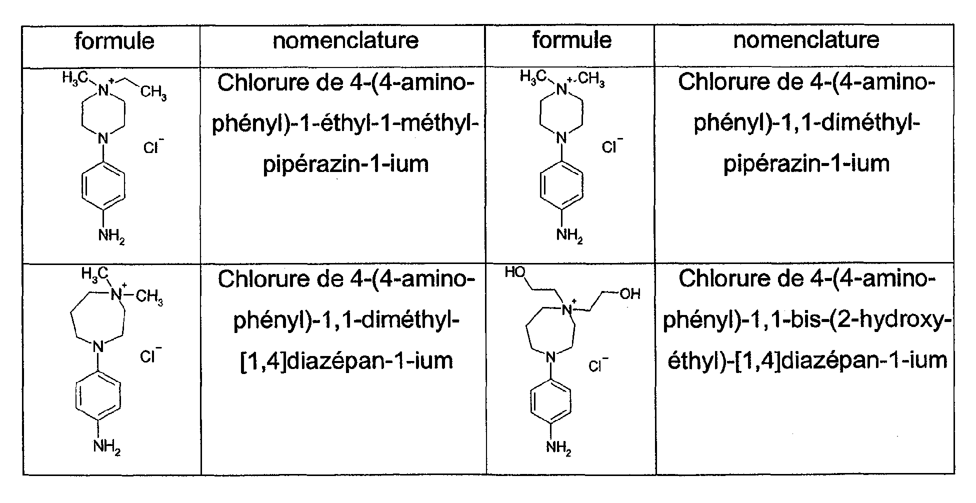

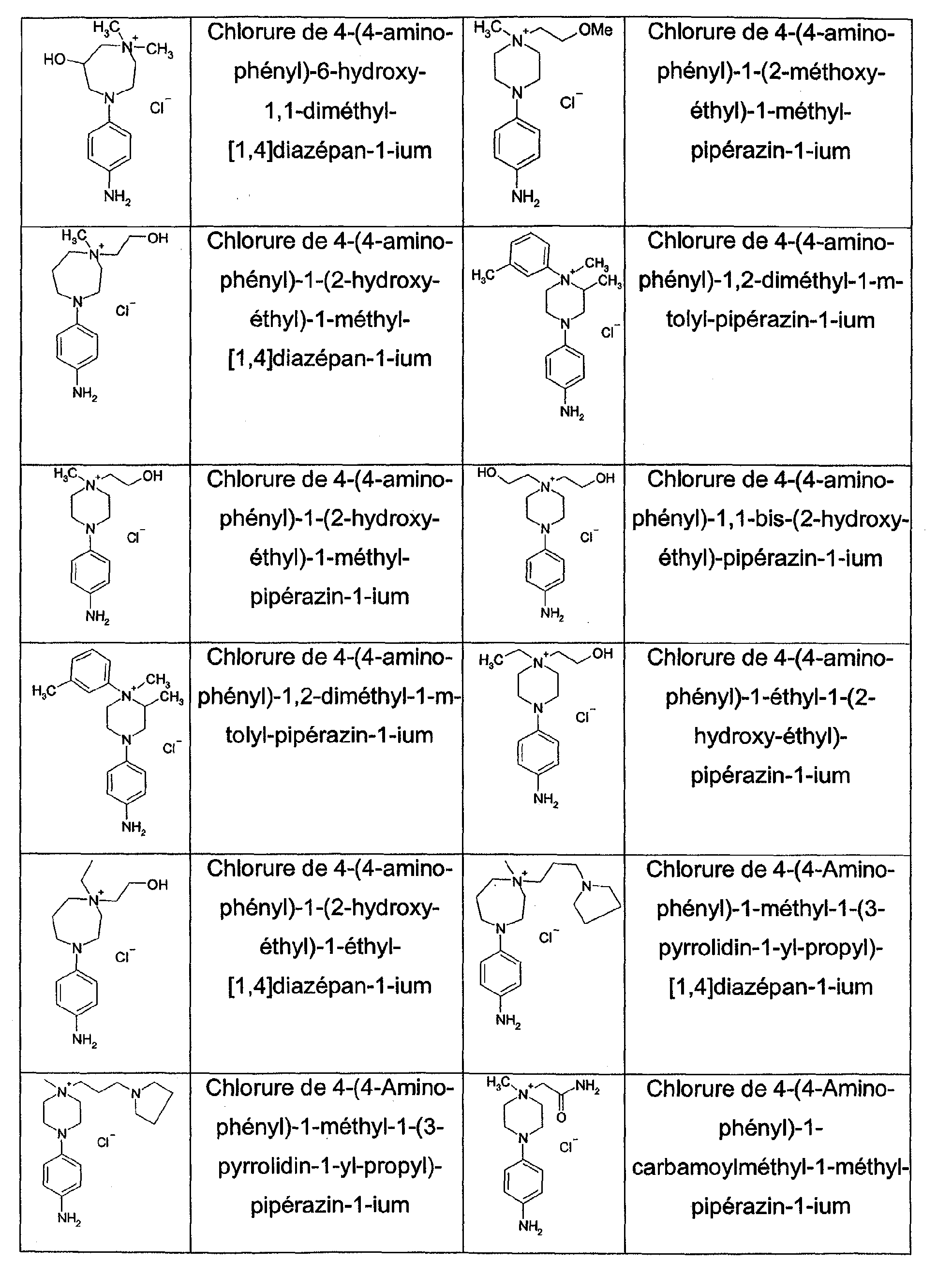

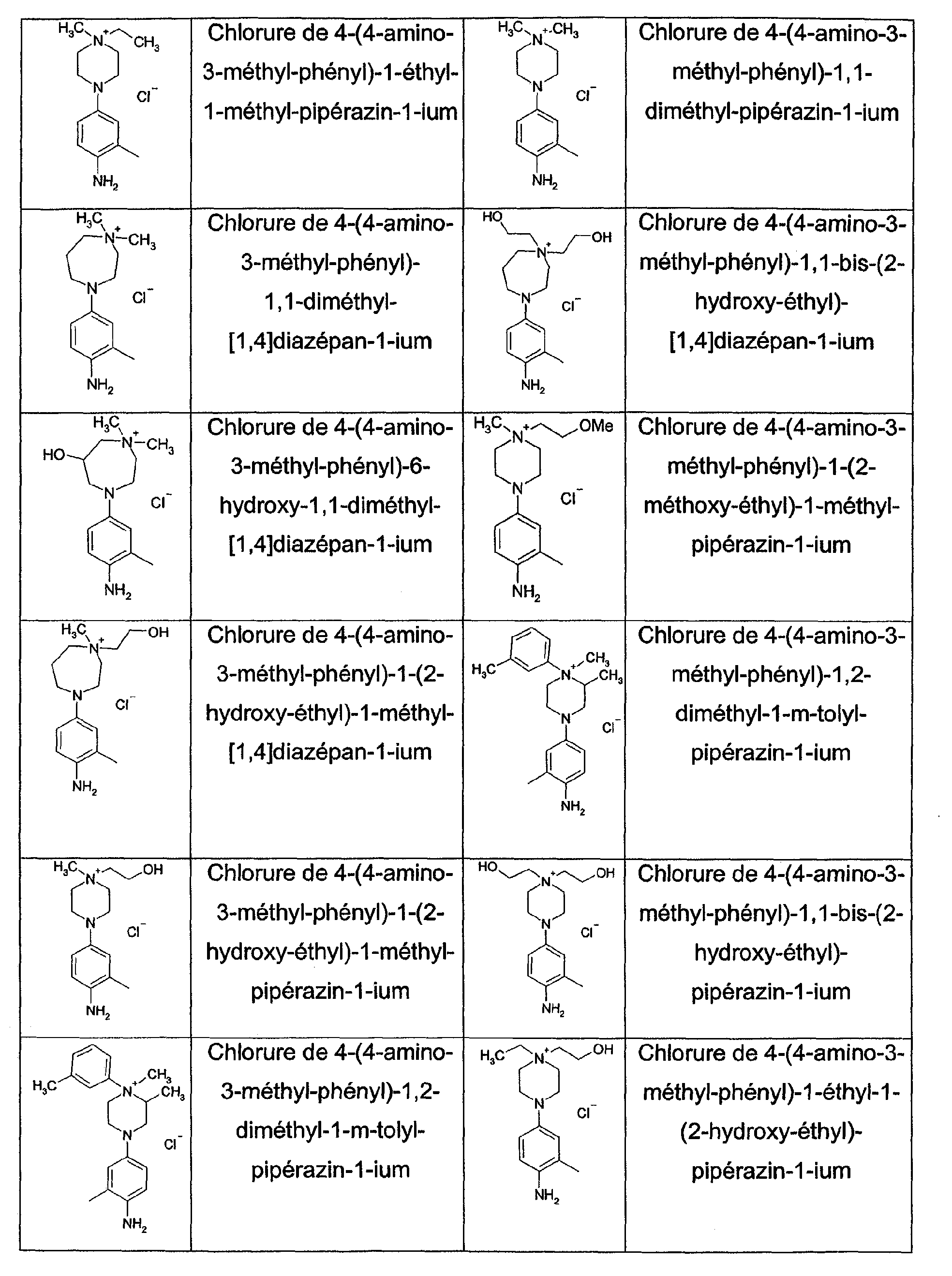

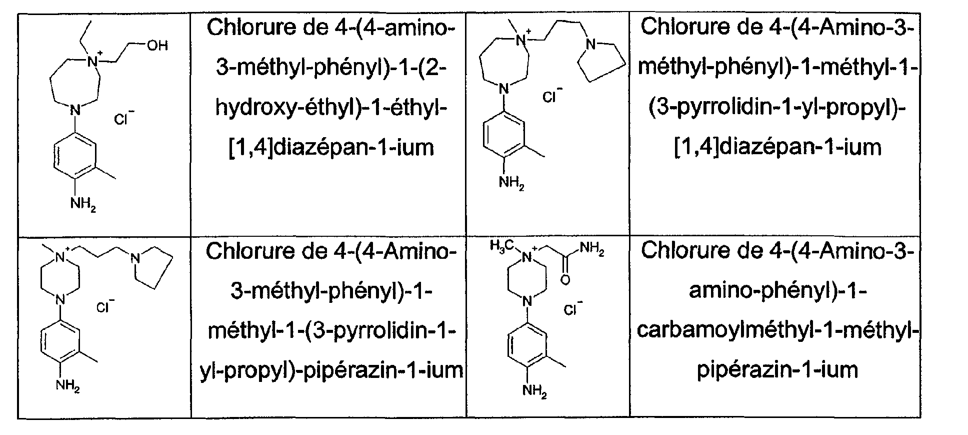

Parmi ces composés, les dérivés de formule (I) particulièrement préférés sont le chlorure de 4-(4-amino-phényl)-1,1-diméthyl-[1,4]diazépan-1-ium, le chlorure de 4-(4-amino-phényl)-1,1-diméthyl-pipérazin-1-ium, le chlorure de 4-(4-amino-phényl)-1-(2-hydroxy-éthyl)-1-méthyl-pipérazin-1-ium.

Le carbone substitué par R₃ ou par R₄ peut être de configuration (R) et / ou (S).

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les para-phénylènediamines autres que celles décrites précédemment, les bis-phénylalkylènediamines, les para-aminophénols, les bis-para-aminophénols, les ortho-aminophénols, les ortho-phénylènediamines, les bases hétérocycliques et leurs sels d'addition.

Parmi les para-phénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les demandes de brevet GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthylamino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3- ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les demandes de brevet DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1 ,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les demandes de brevet DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 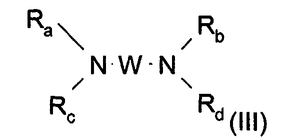 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.
La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La présente invention a également pour objet l'utilisation pour la teinture d'oxydation des fibres kératiniques, et en particulier des fibres kératiniques humaines telles que les cheveux, d'un dérivé de paraphénylènediamine de formule (I) tel que défini précédemment.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentielle ment à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

L'invention a aussi pour objet les dérivés de paraphénylènediamine de formule (I), à l'exception du dérivé de paraphénylènediamine substitué par un cycle diazacyclohexane dans lequel n est égal à 0, m est égal à 0 et R₂ et R'₂ représentent un radical méthyle, ainsi que leurs sels d'addition.

Les dérivés de para-phénylènediamine de formule (I) sont obtenus par réduction des dérivés de para-nitroaniline de formule (II) suivante : 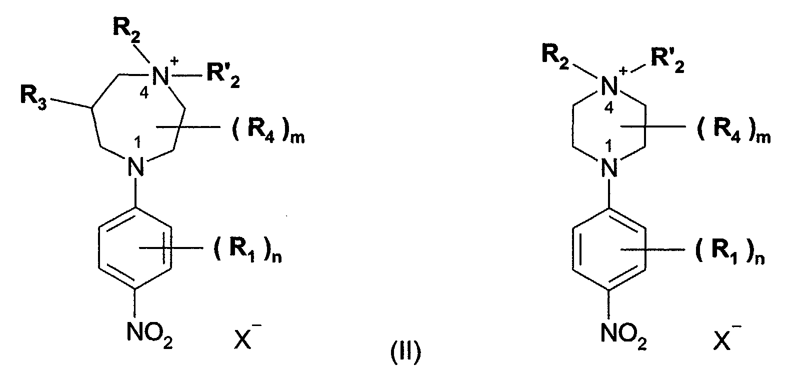 dans laquelle les radicaux R₁, R₂, R'₂, R₃ et R₄ et les entiers n et m sont tels que définis précédemment.

L'invention a aussi pour objet les dérivés de para-nitroaniline de formule (II), à l'exception du 4-(4-nitro-phényl)-1,1-diméthyl-pipérazin-1-ium.

Les exemples qui suivent servent à illustrer l'invention sans toutefois présenter un caractère limitatif.

### EXEMPLES

### Exemple 1 : synthèse du 4-(4-amino-phényl)-1,1-diméthyl-[1,4]diazépan-1-ium, chlorure, dichlorhydrate (4)

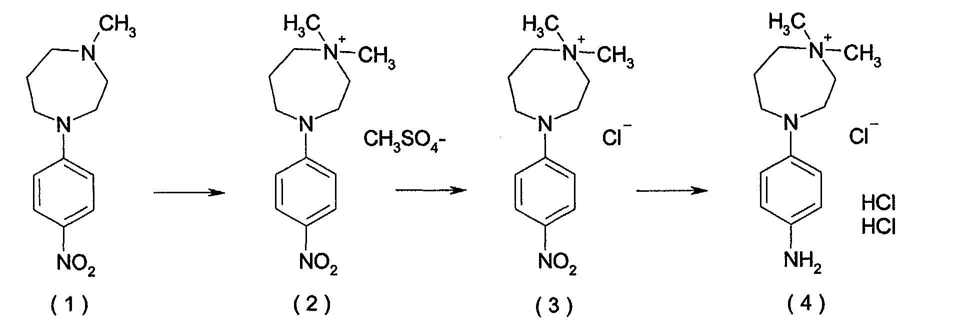

### Synthèse du 1,1-diméthyl-4-(4-nitro-phényl)-[1,4]diazépan-1-ium, méthylsulfate (2)

Dans un réacteur, 23,5 g (0,1 mole) de 1-méthyl-4-(4-nitro-phényl)-[1,4]diazepane (1) sont mis en solution dans 400 ml d'acétate d'éthyle à température ambiante.
11,4 ml (0,12 mole) de sulfate de méthyle sont ajoutés goutte à goutte puis l'agitation est maintenue à température ambiante pendant quatre heures. La réaction est exothermique (28 °C) et un précipité jaune cristallisé se forme.
Le solide est filtré sous vide puis recristallisé dans l'acétate d'éthyle. Les cristaux obtenus sont séchés sous vide à 40 °C sur anhydride phosphorique. 35,6 g de cristaux jaunes sont obtenus (rendement = 98 %).
L'analyse élémentaire pour C₁₄H₂₃N₃SO₄ est la suivante :

| | C % | H % | N % | O % | S% |
|---|---|---|---|---|---|
| Calculé | 46,53 | 6,41 | 11,63 | 26,56 | 8,87 |
| Trouvé | 46,23 | 6,42 | 11,64 | 26,77 | 8,88 |

### Synthèse du 1,1-diméthyl-4-(4-nitro-phényl)-[1,4]diazépan-1-ium, chlorure (3)

L'échange d'anion est réalisé par passage d'une solution de 35,2 g (0,0974 mole) de 1,1-diméthyl-4-(4-nitro-phényl)-[1,4]diazépan-1-ium, méthylsulfate (2) obtenu ci-dessus à l'étape précédente dans un mélange eau/éthanol sur 190 g de résine Amberlite IRA402.

Après évaporation à sec sous pression réduite et recristallisation dans 120 ml d'éthanol 96, 26,4 g (rendement = 95 %) d'un composé cristallisé jaune sont obtenus.
Les résultats obtenus par RMN ¹H sont les suivants :
¹H (400 MHz, DMSO, D6) : 2,31 (m, 2H), 3,25 (s, 6H), 3,6 (m, 2H), 3,65 (m, 2H), 3,72 (m, 2H), 3,97 (m 2H), 6,93 (m, 2H), 8,07 (m, 2H)

### Synthèse du 4-(4-amino-phényl)-1,1-diméthyl-[1,4]diazépan-1-ium, chlorure, dichlorhydrate (4)

Dans un hydrogénateur d'un litre sont introduits 22,9 g (0,08 mole) de 1,1-diméthyl-4-(4-nitro-phényl)-[1,4]diazépan-1-ium, chlorure (3) obtenu ci-dessus à l'étape précédente, 6 g de palladium sur charbon (contenant 50 % d'eau), 400 ml d'éthanol à 96 et 150 ml d'eau.
La réduction se fait en une heure sous une pression d'hydrogène d'environ cinq bars et à une température de 55 °C.
Après filtration du catalyseur sous azote, le milieu réactionnel est versé sur de l'acide chlorhydrique aqueux. Le filtrat est évaporé à sec sous pression réduite. Après recristallisation dans un mélange d'éthanol, acide chlorhydrique et eau et séchage à 45 °C sous vide et sur potasse, 17,8 g de cristaux blancs (rendement = 67 %).
L'analyse élémentaire pour C₁₃H₂₄N₃Cl₃ + H₂O est la suivante :

| | C % | H % | N % | O % | Cl% |
|---|---|---|---|---|---|
| Calculé | 45,03 | 7,56 | 12,12 | 4,61 | 30,68 |
| Trouvé | 45,19 | 7,58 | 12,14 | 4,67 | 30,19 |

Les résultats obtenus par RMN ¹H sont les suivants :
¹H (400 MHz, D₂O): 2,23 (m, 2H), 3,11 (s, 6H), 3,5 (m ,2H), 3,54 (m, 2H),
3,64 (m,2H), 3,78 (m, 2H), 6,99 (m, 2H), 7,3 (m, 2H).

### Exemple 2 : synthèse du 4-(4-aminophényl)-1,1-diméthyl-pipérazin-1-ium, chlorure, dichlorhydrate (4)

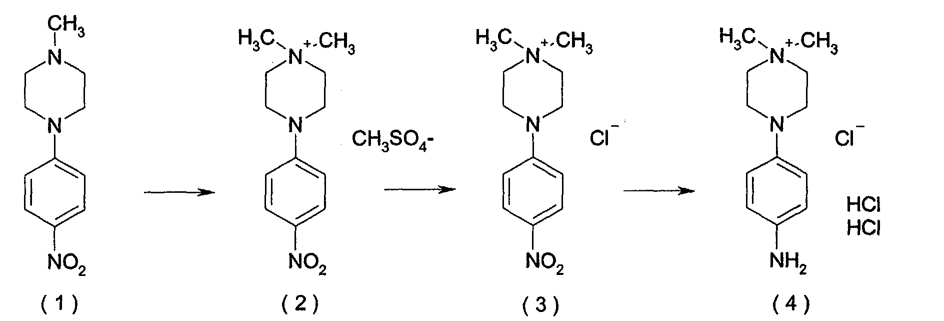

### Synthèse du 1,1-diméthyl-4-(4-nitro-phényl)-pipérazin-1-ium, méthylsulfate (2)

Dans un réacteur, 22,1 g (0,1 mole) de 1-méthyl-4-(4-nitro-phényl)-pipérazine (1) sont mis en solution dans 400 ml d'acétate d'éthyle à température ambiante.
Sous agitation sont ajoutés goutte à goutte 11,4 ml (0,12 mole) de sulfate de méthyle puis l'agitation est maintenue à température ambiante pendant quatre heures. Le précipité jaune formé est filtré sous vide, recristallisé dans l'acétate d'éthyle et séché sous vide à 35 °C sur anhydride phosphorique. 34,3 g de cristaux jaunes sont obtenus (rendement = 99 %).

### Synthèse du 1,1-diméthyl-4-(4-nitro-phényl)-pipérazin-1-ium, chlorure (3)

L'échange d'anion est réalisé par passage d'une solution de 33,0 g (0,095 mole) de 1,1-diméthyl-4-(4-nitro-phényl)-pipérazin-1-ium ; méthylsulfate (2) obtenu ci-dessus à l'étape précédente dans un mélange eau/éthanol sur 120 g de résine Amberlite IRA402.
Après évaporation à sec sous pression réduite, 20,0 g d'un composé cristallisé jaune sont obtenus (rendement = 77 %).
Les résultats obtenus par RMN ¹H sont les suivants :
¹H RMN (400 MHz, DMSO, D6) : 3,235 (s, 6H, N-(CH₃)₂) ; 3,37 ( s, 3H, CH₃OSO₃-); 3,58 (m, 4H, CH₂) ; 3,829 (m, 4H, CH₂) ; 7,15 (d, 2 H ,ortho de N) ; 8,14 (d, 2 H, ortho de NO₂).

### Synthèse du 4-(4-amino-phényl)-1,1-diméthyl-pipérazin-1-ium, chlorure, dichlorhydrate (4)

Dans un hydrogénateur d'un litre sont placés 19,0 g (0,07 mole) 1,1-diméthyl-4-(4-nitro-phényl)-pipérazin-1-ium, chlorure (3) obtenu ci-dessus à l'étape précédente, 5 g de palladium sur charbon (contenant 50 % d'eau), 400 ml d'éthanol à 96 et 150 ml d'eau.
La réduction se fait en une heure et demi sous une pression d'hydrogène d'environ six bars et à une température de 50 °C.
Après filtration du catalyseur sous azote, le milieu réactionnel est versé sur de l'acide chlorhydrique aqueux. Le filtrat est évaporé à sec sous pression réduite. Après séchage à 45 °C sous vide et sur potasse, 20,2 g de cristaux blancs sont obtenus (rendement = 67 %).
L'analyse élémentaire pour C₁₂H₂₂N₃Cl₃ + 1,5H₂O est la suivante :

| | C% | H % | N % | O% | Cl% |
|---|---|---|---|---|---|
| Calculé : | 42,18 | 7,37 | 12,30 | 7,02 | 31,13 |
| Trouvé : | 42,46 | 7,14 | 12,43 | 7,86 | 30,09 |

Les résultats obtenus par RMN ¹H sont les suivants :
¹H RMN (400 MHz, D₂O) : 3,33 (s, 6H, CH₃); 3,71 (m, 8H, CH₂); 7,32 (m, 2H, ortho de N); 7,49 (m, 2H, ortho de NH₂).

### Exemple 3 : synthèse du 4-(4-amino-phényl)-1-(2-hydroxy-éthyl)-1-méthyl-pipérazin-1-ium, chlorure, dichlorhydrate (4)

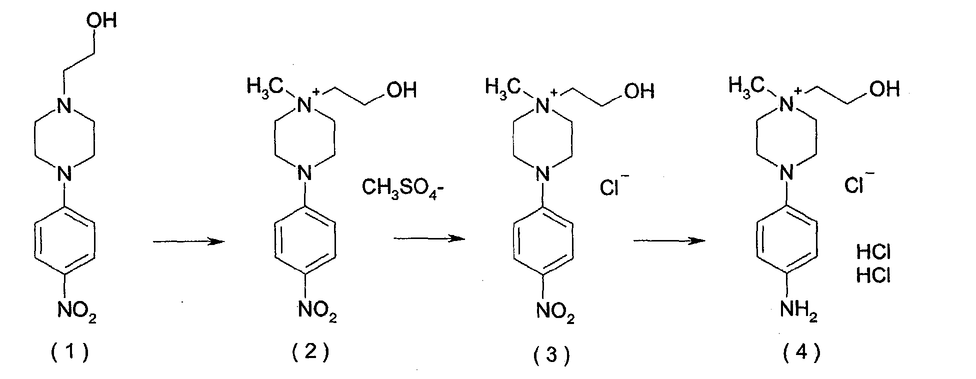

### Synthèse du 1-(2-hydroxy-éthyl)-1-méthyl-4-(4-nitro-phényl)-pipérazin-1-ium méthylsulfate (2),

Dans un réacteur, 25,1 g (0,1 mole) de 2-[4-(4-nitro-phényl)-pipérazin-1-yl]-éthanol (1) sont mis en solution dans 500 ml d'acétate d'éthyle chauffé à 40 °C.
Sous agitation sont ajoutés goutte à goutte 11,4 ml (0,12 mole) de sulfate de méthyle puis l'agitation est maintenue à température ambiante pendant cinq heures. Le précipité jaune formé est filtré sous vide, recristallisé dans l'acétate d'éthyle et séché sous vide à 40 °C sur anhydride phosphorique. 38,0 g (rendement quantitatif) de cristaux jaunes sont obtenus.
L'analyse élémentaire pour C₁₄H₂₃N₃SO₇ est la suivante :

| | C % | H % | N % | O % | S% |
|---|---|---|---|---|---|
| Calculé | 44,55 | 6,14 | 11,13 | 29,67 | 8,50 |
| Trouvé | 44,63 | 6,14 | 11,20 | 29,55 | 8,56 |

### Synthèse du 1-(2-hydroxy-éthyl)-1-méthyl-4-(4-nitro-phényl)-pipérazin-1-ium, chlorure (3)

L'échange d'anion est réalisé par passage d'une solution de 38,0 g (0,1 mole) de 1-(2-hydroxy-éthyl)-1-méthyl-4-(4-nitro-phényl)-pipérazin-1-ium, méthylsulfate (2) obtenu ci-dessus à l'étape précédente dans un mélange eau/éthanol sur 190 g de résine Amberlite IRA402
Après évaporation à sec sous pression réduite et recristallisation de 220 ml d'éthanol 96 au reflux, 27,0 g d'un composé cristallisé jaune sont obtenus (rendement = 71 %).
Les résultats obtenus par RMN ¹H sont les suivants :
¹H RMN (400 MHz, DMSO, D6): 3,25 (s, 3H, CH₃N) ; 3,373 (s, 3H, CH₃OSO₃-) ; 3,615- 3,925 (m, 12H, CH₂) ; 5,52 (t, 1H, OH) ; 7,148 (d, 2 H, ortho de N); 8,146 (d, 2 H, ortho de NO₂).

### Synthèse du 4-(4-amino-phényl)-1-(2-hydroxy-éthyl)-1-méthyl-pipérazin-1-ium, chlorure, dichlorhydrate (4)

Dans un hydrogénateur d'un litre sont placés 14,1 g (0,08 mole) de 1-(2-hydroxy-éthyl)-1-méthyl-4-(4-nitro-phényl)-pipérazin-1-ium, chlorure (3) obtenu ci-dessus à l'étape précédente, 6 g de palladium sur charbon (contenant 50 % d'eau), 400 ml d'éthanol à 96 et 150 ml d'eau.
La réduction se fait en deux heures sous une pression d'hydrogène d'environ cinq bars et à une température de 50 °C.
Après filtration du catalyseur sous azote, le milieu réactionnel est versé sur de l'acide chlorhydrique aqueux. Le filtrat est évaporé à sec sous pression réduite. Après séchage à 45 °C sous vide et sur potasse, 23,2 g de cristaux blancs sont obtenus (rendement = 80 %).
Les résultats obtenus par RMN ¹H sont les suivants :
¹H RMN (400 MHz, DMSO, D6) : 3,24 (s, 3H, CH₃) ; 3,53 (m, 10H, CH₂); 3,87 (m, 2H, CH₂); 7,08 (d, 2H, ortho de N) ; 7,31 (d, 2H, ortho de NH₂); 9,25 (m, 1H, OH); 10,52 (m, 2H, NH₂).

### EXEMPLES DE TEINTURE

### EXEMPLES 1 A 5 : Teinture en milieu acide à partir du 4-(4-aminophényl)-1,1-diméthyl-[1,4]diazépan-1-ium, chlorure, dichlorhydrate

Les compositions tinctoriales suivantes ont été préparées :

| **Exemples** | **1** | **2** | **3** | **4** | **5** |
|---|---|---|---|---|---|
| 4-(4-amino-phényl)-1,1-diméthyl-[1,4]diazépan-1-ium ; chlorure ; dichlorhydrate (base) | 10⁻³ mole | 10⁻³ mole | 10⁻³ mole | 10⁻³ mole | 10⁻³ mole |
| 2-(2,4-Diamino-phénoxy)-éthanol, dichlorhydrate (coupleur) | 10⁻³ mole - | | - | | - |
| 3-Amino-2-chloro-6-méthylphénol, chlorhydrate -(coupleur) | | 10⁻³ mole - | | | - |
| 2-méthyl-5-aminophénol (coupleur) | - | - | 10⁻³ mole | | |
| 2-amino-pyridin-3-ol (coupleur) - | | - | - | 10⁻³ mole | |
| 1H-indol-6-ol (coupleur) | | | | | 10⁻³ mole |
| Support de teinture (2) | (*) | (*) | (*) | (*) | (*) |
| Eau déminéralisée q.s.p. | 100 g | 100 g | 100 g | 100 g | 100 g |
| (*) Support de teinture (2) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7. Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **1** | **2** | **3** | **4** | **5** |
|---|---|---|---|---|---|
| Nuance observée | Bleu violet | Violet | Beige violet | Beige violet | Beige |

### EXEMPLES 6 A 8 : Teinture en milieu alcalin à partir du 4-(4-aminophényl)-1,1-diméthyl-[1,4]diazépan-1-ium, chlorure, dichlorhydrate

Les compositions tinctoriales suivantes ont été préparées :

| **Exemples** | **6** | **7** | **8** |
|---|---|---|---|
| 4-(4-amino-phényl)-1,1-diméthyl-[1,4]diazépan-1-ium ; chlorure ; dichlorhydrate (base) | 10⁻³ mole | 10⁻³ mole | 10⁻³ mole |
| 2-(2,4-Diamino-phénoxy)-éthanol, dichlorhydrate (coupleur) | 10⁻³ mole | - | - |
| 4,6-diméthyl-2H-pyrazolo[3,2c] -[1,2,4] triazole (coupleur) | | 10⁻³ Mole - | |
| 3-Amino-2-chloro-6-méthylphénol, chlorhydrate (coupleur) | - | - | 10⁻³ mole |
| Support de teinture (1) | (*) | (*) | (*) |
| Eau déminéralisée q.s.p. | 100 g | 100 g | 100 g |
| (*) Support de teinture (1) pH 9,5 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5. Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **6** | **7** | **8** |
|---|---|---|---|
| Nuance observée | Bleu violet | Violet rouge | Violet |

### EXEMPLE 9 A 11 : Teinture en milieu acide à partir du du 4-(4-aminophényl)-1,1-diméthyl-pipérazin-1-ium ; chlorure ; dichlorhydrate

Les compositions tinctoriales suivantes ont été préparées :

| **Exemples** | **9** | **10** | **11** |
|---|---|---|---|
| 4-(4-amino-phényl)-1,1-diméthyl-pipérazin-1-ium ; chlorure ; dichlorhydrate (base) | 10⁻³ mole | 10⁻³ mole | 10⁻³ mole |
| 2-(2,4-diamino-phénoxy)-éthanol ; dichlorhydrate (coupleur) | 10⁻³ Mole | | - |
| 2-amino-pyridin-3-ol (coupleur) | - | 10⁻³ mole - | |
| 1 H-indol-6-ol (coupleur) | - | - | 10⁻³ mole |
| Support de teinture (2) | (*) | (*) | (*) |
| Eau déminéralisée q.s.p. | 100 g | 100 g | 100 g |
| (*) Support de teinture (2) pH 7 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **9** | **10** | **11** |
|---|---|---|---|
| Nuance observée | Beige | Orangé | Gris orangé |

### EXEMPLE 12 : Teinture en milieu alcalin à partir du 4-(4-amino-phényl)-1,1-diméthyl-pipérazin-1-ium ; chlorure ; dichlorhydrate

Les compositions tinctoriales suivantes ont été préparées :

| **Exemples** | **12** |
|---|---|
| 4-(4-amino-phényl)-1,1-diméthyl-pipérazin-1-ium ; chlorure ; dichlorhydrate (base d'oxydation) | 10⁻³ mole |
| 2-(2,4-Diamino-phénoxy)-éthanol, dichlorhydrate (coupleur) | 10⁻³ mole |
| Support de teinture (1) | (*) |
| Eau déminéralisée q.s.p. | 100 g |
| (*) Support de teinture (1) pH 9,5 | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5. Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **12** |
|---|---|
| Nuance observée | Beige |

### EXEMPLES 13 A 15 : Teinture en milieu acide à partir du 4-(4-aminophényl)-1-(2-hydroxy-éthyl)-1-méthyl-pipérazin-1-ium ; chlorure ; dichlorhydrate

Les compositions tinctoriales suivantes ont été préparées :

| **Exemples** | **13** | **14** | **15** |
|---|---|---|---|
| 4-(4-amino-phényl)-1-(2-hydroxy-éthyl)-1-méthyl-pipérazin-1-ium ; chlorure ; dichlorhydrate (base) | 10⁻³ mole | 10⁻³ mole | 10⁻³ mole |
| 2-(2,4-Diamino-phénoxy)-éthanol, dichlorhydrate (coupleur) | 10⁻³ mole - | | - |
| 2-amino-pyridin-3-ol (coupleur) | - | 10⁻³ mole | - |
| 1 H-indol-6-ol (coupleur) | - | - | 10⁻³ mole |
| Support de teinture (2) | (*) | (*) | (*) |
| Eau déminéralisée q.s.p. | 100 g | 100 g | 100 g |
| (*) Support de teinture (2) pH 7 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7. Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **13** | **14** | **15** |
|---|---|---|---|
| Nuance observée | Beige | Orangé | Gris orangé |

### EXEMPLE 16 : Teinture en milieu alcalin à partir 4-(4-amino-phényl)-1- (2-hydroxy-éthyl)-1-méthyl-pipérazin-1-ium ; chlorure ; dichlorhydrate

Les compositions tinctoriales suivantes ont été préparées :

| **Exemples** | **16** |
|---|---|
| 4-(4-amino-phényl)-1-(2-hydroxy-éthyl)-1-méthyl-pipérazin-1-ium ; chlorure ; dichlorhydrate (base) | 10⁻³ mole |
| 2-(2,4-Diamino-phénoxy)-éthanol, dichlorhydrate (coupleur) | 10⁻³ mole |
| Support de teinture (1) | (*) |
| Eau déminéralisée q.s.p. | 100 g |
| (*) Support de teinture (1) pH 9,5 | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5. Chaque mélange obtenu est appliqué sur des mèches de cheveux gris à 90 % de blancs. Après 30 min de pose, les mèches sont rinçées, lavées avec un shampooing standard, rinçées à nouveau puis séchées.
Les résultats de teinture suivants ont été obtenus.

| **Exemples** | **16** |
|---|---|
| Nuance observée | Beige |

## Revendications

1. Composition tinctoriale des fibres kératiniques comprenant, dans un milieu cosmétique approprié à la teinture, à titre de base d'oxydation, au moins un dérivé de para-phénylènediamine de formule (I) ou un de ses sels d'addition : 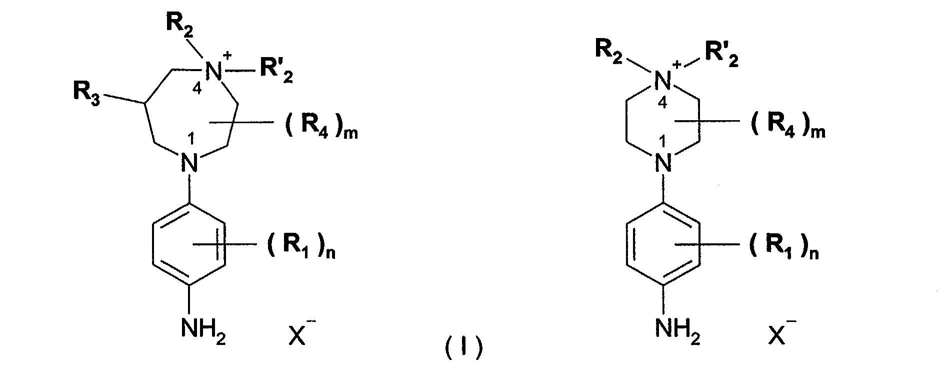 dans laquelle :
• R₁ représente :
- un atome d'halogène ;
- une chaîne hydrocarbonée en C₁-C₈, aliphatique ou alicyclique, saturée ou insaturée, un ou plusieurs atomes de carbone pouvant être remplacés par un ou plusieurs atomes d'oxygène, d'azote, de silicium, de soufre ou groupements SO₂; le radical R₁ ne comportant pas de liaison peroxyde, ni de radicaux diazo, nitro ou nitroso ;
• n est compris entre 0 et 4, étant entendu que lorsque n est supérieu à 1 alors les radicaux R₁ peuvent être identiques ou différents ;
• R₂ et R'₂, identiques ou différents, représentent :
- un radical alkyle pouvant être insaturé, non substitué ou substitué par un ou plusieurs radicaux carboxyle, alkylcarbonyle, alcoxycarbonyle, carbamoyle, mono- ou di-alkylcarbamoyle, hétérocycliques à 4, 5, 6 ou 7 atomes, saturés ou insaturés, dont le ou les hétéroatomes sont choisis parmi l'azote, l'oxygène et le soufre ;
- un radical -CH₂ R où R est un radical alkyle pouvant être insaturé, substitué par un ou plusieurs radicaux hydroxy, alcoxy, hydrogénothio, halogéno, amino, mono- ou di-alkylamino, amino avec l'amine substituée par un radical alkylcarbonyle, carbamyle ou alkylsulfonyle ;
- un radical aryle ;
- un radical benzyle ;
• R₃ représente :
- un atome d'hydrogène ;
- un radical alkyle pouvant être insaturé ;
- un radical hydroxy ;
- un radical hydroxyalkyle ;
- un radical alcoxy ;
- un radical alcoxyalkyle ;
- un radical alkylcarbonyle ;
- un radical hydroxyalcoxyalkyle ;
- un radical amino, l'amine pouvant être mono ou disubstituée par un radical alkyle, acétyle ou hydroxyalkyle ;
- un radical monoalkylamino ou dialkylamino ;
- un radical aminoalkyle, l'amine pouvant être mono ou disubstituée par un radical alkyle, acétyle ou hydroxyalkyle ;
- un radical hydroxy- et amino- alkyle ;
- un radical carboxyle ;
- un radical carboxyalkyle ;
- un radical carbamoyle ;
- un radical carbamoylalkyle ;
- un radical alcoxycarbonyle ;
- un radical mono- ou dialkyl- aminocarbonyle ;
- un radical mono- ou dialkyl- aminocarbonylalkyle ;
• R₄ représente :
- un radical alkyle pouvant être insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine pouvant être mono ou disubstituée par un radical alkyle, acétyle, hydroxyalkyle ;
- un radical hydroxy- et amino- alkyle ;
- un radical carboxyle ;
- un radical carboxyalkyle ;
- un radical carbamoyle ;
- un radical carbamoylalkyle ;
- un radical alcoxycarbonyle ;
- un radical mono- ou dialkyl- aminocarbonyle ;
- un radical mono- ou dialkyl- aminocarbonylalkyle ;
• m est compris entre 0 et 4, étant entendu que lorsque m est supérieur à 1 alors les radicaux R₄ peuvent être identiques ou différents ;
• X⁻ représente un contre-ion.

2. Composition selon la revendication 1 dans laquelle n est égal à 0 ou R₁ est choisi parmi un radical alkyle ; un radical hydroxyalkyle ; un radical aminoalkyle ; un radical alcoxy ; un radical hydroxyalcoxy.

3. Composition selon la revendication 2 dans laquelle n est égal à 0 ou R₁ est choisi parmi un radical méthyle ; un radical hydroxyméthyle ; un radical 2-hydroxyéthyle ; un radical 1,2-dihydroxyéthyle ; un radical méthoxy ; un radical isopropyloxy ; un radical 2-hydroxyéthoxy.

4. Composition selon l'une quelconque des revendications précédentes dans laquelle n est égal à 0 ou à 1.

5. Composition selon l'une quelconque des revendications précédentes dans laquelle R₂ est choisi parmi un radical alkyle ; un radical alkyle substitué par un radical hétérocyclique à 4, 5, 6 ou 7 atomes, saturé ou insaturé, dont le ou les hétéroatomes sont choisis parmi l'azote, l'oxygène et le soufre; un radical -CH₂R où R est un radical alkyle substitué par un ou plusieurs radicaux hydroxy.

6. Composition selon la revendication 5 dans laquelle R₂ est choisi parmi un radical 2-hydroxyéthyle ; un radical 2,3-dihydroxypropyle ; un radical 3-(1-pyrrolidinyl)propyle ; un radical méthyle.

7. Composition selon l'une quelconque des revendications précédentes dans laquelle R'₂ est choisi parmi un radical alkyle ; un radical -CH₂R où R est un radical alkyle substitué par un ou plusieurs hydroxy, amino, mono ou dialkylamino.

8. Composition selon la revendication 7 dans lesquels R'₂ est choisi parmi un radical méthyle ; un radical éthyle ; un radical 2-hydroxyéthyle.

9. Composition selon l'une quelconque des revendications précédentes dans laquelle R₃ est choisi parmi un atome d'hydrogène ; un radical alkyle ; un radical hydroxyalkyle ; un radical aminoalkyle ; un radical carboxyle ; un radical carbamoyle ; un radical hydroxyle ; un radical alcoxy ; un radical amino ; un radical mono ou dialkylamino.

10. Composition selon la revendication 9 dans laquelle R₃ est choisi parmi un atome d'hydrogène ; un radical hydroxyle ; un radical carboxyle ; un radical carbamoyle ; un radical amino ; un radical hydroxyméthyle ; un radical aminométhyle.

11. Composition selon l'une quelconque des revendications précédentes dans laquelle m est égal à 0 ou R₄ est choisi parmi un radical alkyle ; un radical hydroxyalkyle ; un radical aminoalkyle ; un radical carboxyle ; un radical carbamoyle ; un radical mono ou dialkylcarbamoyle.

12. Composition selon la revendication 11 dans laquelle m est égal à 0 ou 1.

13. Composition selon l'une quelconque des revendications précédentes dans laquelle le composé de formule (I) est choisi parmi le chlorure de 4-(4-amino-phényl)-1-éthyl-1-méthyl-pipérazin-1-ium ; le chlorure de 4-(4-amino-phényl)-1,1-diméthyl-pipérazin-1-ium ; le chlorure de 4-(4-amino-phényl)-1,1-diméthyl-[1,4]diazépan-1-ium ; le chlorure de 4-(4-aminophényl)-1,1-bis-(2-hydroxy-éthyl)-[1,4]diazépan-1-ium ; le chlorure de 4-(4-amino-phényl)-6-hydroxy-1,1-diméthyl-[1,4]diazépan-1-ium ; le chlorure de 4-(4-amino-phényl)-1-(2-méthoxy-éthyl)-1-méthyl-pipérazin-1-ium ; le chlorure de 4-(4-amino-phényl)-1-(2-hydroxy-éthyl)-1-méthyl-[1,4]diazépan-1-ium ; le chlorure de 4-(4-amino-phényl)-1,2-diméthyl-1-m-tolyl-pipérazin-1-ium ; le chlorure de 4-(4-amino-phényl)-1-(2-hydroxy-éthyl)-1-méthyl-pipérazin-1-ium ; le chlorure de 4-(4-amino-phényl)-1,1-bis-(2-hydroxy-éthyl)-pipérazin-1-ium ; le chlorure de 4-(4-amino-phényl)-1 ,2-diméthyl-1-m-tolyl-pipérazin-1-ium ; le chlorure de 4-(4-amino-phényl)-1-éthyl-1-(2-hydroxy-éthyl)-pipérazin-1-ium ; le chlorure de 4-(4-amino-phényl)-1-(2-hydroxy-éthyl)-1-éthyl-[1,4]diazépan-1-ium ; le chlorure de 4-(4-amino-phényl)-1-méthyl-1-(3-pyrrolidin-1-yl-propyl)-[1,4]diazépan-1-ium ; le chlorure de 4-(4-amino-phényl)-1-méthyl-1-(3-pyrrolidin-1-yl-propyl)-pipérazin-1-ium ; le chlorure de 4-(4-amino-phényl)-1-carbamoylméthyl-1-méthyl-pipérazin-1-ium ; le chlorure de 4-(4-amino-3-méthyl-phényl)-1-éthyl-1-méthyl-pipérazin-1-ium ; le chlorure de 4-(4-amino-3-méthyl-phényl)-1,1-diméthyl-pipérazin-1-ium ; le chlorure de 4-(4-amino-3-méthyl-phényl)-1,1-diméthyl-[1,4]diazépan-1-ium ; le chlorure de 4-(4-amino-3-méthyl-phényl)-1,1-bis-(2-hydroxy-éthyl)-[1,4]diazépan-1-ium ; le chlorure de 4-(4-amino-3-méthyl-phényl)-6-hydroxy-1,1-diméthyl-[1,4]diazépan-1-ium ; le chlorure de 4-(4-amino-3-méthyl-phényl)-1-(2-méthoxy-éthyl)-1-méthyl-pipérazin-1-ium ; le chlorure de 4-(4-amino-3-méthyl-phényl)-1-(2-hydroxy-éthyl)-1-méthyl-[1,4]diazépan-1-ium ; le chlorure de 4-(4-amino-3-méthyl-phényl)-1,2-diméthyl-1-m-tolyl-pipérazin-1-ium ; le chlorure de 4-(4-amino-3-méthyl-phényl)-1-(2-hydroxy-éthyl)-1-méthyl-pipérazin-1-ium ; le chlorure de 4-(4-amino-3-méthy)-phényl)-1,1-bis-(2-hydroxy-éthyl)-pipérazin-1-ium ; le chlorure de 4-(4-amino-3-méthyl-phényl)-1,2-diméthyl-1-m-tolyl-pipérazin-1-ium ; le chlorure de 4-(4-amino-3-méthyl-phényl)-1-éthyl-1-(2-hydroxy-éthyl)-pipérazin-1-ium ; le chlorure de 4-(4-amino-3-méthyl-phényl)-1-(2-hydroxy-éthyl)-1-éthyl-[1,4]diazépan-1-ium ; le chlorure de 4-(4-amino-3-méthyl-phényl)-1-méthyl-1-(3-pyrrolidin-1-yl-propyl)-[1 ,4]diazépan-1-ium ; le chlorure de 4-(4-amino-3-méthyl-phényl)-1-méthyl-1-(3-pyrrolidin-1-yl-propyl)-pipérazin-1-ium ; le chlorure de 4-(4-amino-3-amino-phényl)-1-carbamoylméthyl-1-méthyl-pipérazin-1-ium.

14. Composition selon la revendication 13 dans laquelle le composé de formule (I) est choisi parmi le chlorure de 4-(4-amino-phényl)-1,1-diméthyl-[1,4]diazépan-1-ium ; le chlorure de 4-(4-amino-phényl)-1,1-diméthyl-pipérazin-1-ium ; le chlorure de 4-(4-amino-phényl)-1-(2-hydroxy-éthyl)-1-méthyl-pipérazin-1-ium.

15. Composition selon l'une quelconque des revendications précédentes comprenant de plus un coupleur choisi parmi les méta-phénylènediamines, les méta-aminophénols, les méta-diphénols, les coupleurs naphtaléniques, les coupleurs hétérocycliques et leurs sels d'addition.

16. Composition selon la revendication 15 dans laquelle la quantité de chacun des coupleurs est comprise entre 0,001 et 10 % en poids du poids total de la composition tinctoriale, de préférence entre 0,005 et 6 %.

17. Composition selon l'une quelconque des revendications précédentes comprenant une base d'oxydation additionnelle autre que les bases d'oxydation de formule (I) choisie parmi les para-phénylènediamines, les bis-phénylalkylènediamines, les para-aminophénols, les bis-para-aminophénols, les ortho-aminophénols, les ortho-phénylènediamines, les bases hétérocycliques et leurs sels d'addition.

18. Composition selon la revendication 17 dans laquelle la quantité de chacune des bases d'oxydation est comprise entre 0,001 et 10 % en poids du poids total de la composition tinctoriale, de préférence entre 0,005 et 6 %.

19. Utilisation pour la teinture d'oxydation des fibres kératiniques d'un composé de formule (I) tel que défini dans l'une quelconque des revendications 1 à 14.

20. Procédé de teinture des fibres kératiniques, **caractérisé en ce qu'**une composition telle que définie dans l'une quelconque des revendications 1 à 18 est appliquée sur les fibres kératiniques en présence d'un agent oxydant pendant un temps suffisant pour développer la coloration désirée.

21. Procédé selon la revendication 20 dans lequel l'agent oxydant est choisi parmi le peroxyde d'hydrogène, le peroxyde d'urée, les bromates de métaux alcalins, les persels, les peracides et les enzymes oxydases.

22. Dispositif à plusieurs compartiments dans lequel un premier compartiment contient une composition tinctoriale telle que définie dans l'une quelconque des revendications 1 à 18 et un deuxième compartiment contient un agent oxydant.

23. Dérivés de paraphénylènediamine de formule (I) tels que définis dans l'une quelconque des revendications 1 à 14, à l'exception du dérivé de para-phénylènediamine substitué par un cycle diazacyclohexane dans lequel n est égal à 0, m est égal à 0 et R₂ et R'₂ représentent un radical méthyle, ainsi que leurs sels d'addition.

24. Dérivés de para-nitroaniline de formule (II) suivante : 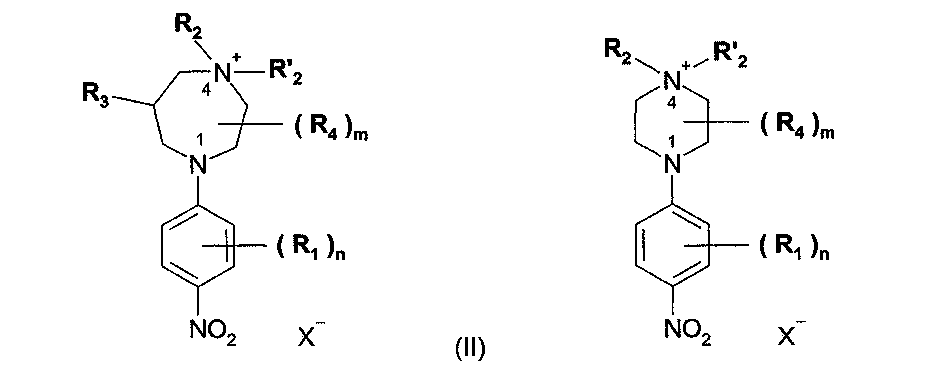 dans laquelle les radicaux R₁, R₂, R'₂, R₃ et R₄ et les entiers n et m sont tels que définis dans les revendications 1 à 14, à l'exception du 4-(4-nitro-phényl)-1,1-diméthyl-pipérazin-1-ium.
